Europäisches Patentamt

European Patent Office

Office européen des brevets

Publication number: **0 274 887**
**A1**

## EUROPEAN PATENT APPLICATION

(21) Application number: 87311155.3

(22) Date of filing: 17.12.87

(51) Int. Cl.4: **C07C 55/10** , C07C 51/44 , C07C 51/42 , C07C 51/573 , C07C 51/56

(30) Priority: 17.12.86 GB 8630104

(43) Date of publication of application: 20.07.88 Bulletin 88/29

(84) Designated Contracting States: BE DE FR GB IT NL

(71) Applicant: **BP Chemicals Limited** Belgrave House 76 Buckingham Palace Road London, SW1W 0SU(GB)

(72) Inventor: **Cooper, Jeremy Bernard BP Chemicals Limited** Belgrave House 76 Buckingham Palace Road London, SW1W 0SU(GB)

(74) Representative: **Richardson, Derek et al BP INTERNATIONAL LIMITED Patents & Agreements Division Chertsey Road Sunbury-on-Thames Middlesex TW16 7LN(GB)**

(54) Process for the recovery of succinic acid from its mixtures with other carboxylic acids.

(57) Succinic acid is recovered from its mixture with other carboxylic acids by a process which comprises the steps of:

(A) heating the mixture of carboxylic acids under conditions of temperature and pressure and for a time sufficient to dehydrate at least a part of the succinic acid to succinic anhydride,

(B) separating by distillation the product from step (A) into a base fraction comprising carboxylic acids having a boiling point greater than that of succinic anhydride and a distillate fraction comprising succinic anhydride and succinic acid formed by hydrolysis of succinic anhydride by water resulting from dehydration in step (A), and

(C) recovering succinic acid from the distillate fraction obtained in step (B).

EP 0 274 887 A1

## PROCESS FOR THE RECOVERY OF SUCCINIC ACID FROM ITS MIXTURES WITH OTHER CARBOXYLIC ACIDS

The present invention relates to the recovery of succinic acid from its mixtures with other carboxylic acids.

Succinic acid is a valuable chemical product which is used in agriculture as a plant growth accelerator and cut-flower preservative, in food as a preservative, in textiles in size preparation and in plastics as a colour stabiliser and cross-linking agent. It is produced as a by-product in admixture with other carboxylic acids and/or oxygenated materials in a number of commercial processes. Thus, the oxidation of cyclohexanol/cyclohexanone mixtures with nitric acid produces adipic acid as primary produce and a mixture of succinic and glutaric acids as by-products. In the liquid-phase oxidation of paraffinic hydrocarbons with molecular oxygen, lower carboxylic acids are produced as the principal products together with numerous by-products, including laevulinic acid, succinic acid, glutaric acid and adipic acid in addition to other high boiling organics including esters, ketones, aldehydes, lactones, polymeric resins and tars, which may form up to 50% by weight or more of the mixture. A desirable objective would be to recover succinic acid from complex mixtures of carboxylic acids, particularly mixtures of the aforesaid type.

A number of processes have been developed for the recovery of succinic acid from its admixtures with other carboxylic acids. One such process involves cooling, crystallisation and centrifugation, followed by upgrading to a saleable product by a relatively complex washing/recrystallisation procedure. Another process, the subject of GB-A-1526073, involves recovering succinic acid of purity greater than 70% w.w from mixtures containing it together with adipic and glutaric acids by contacting the mixture with a liquid ester comprising ethyl, n-butyl or sec-butyl acetate or a mixture thereof at a temperature in the range from 0 to 75°C thereby effecting precipitation of a solid containing more than 70% w/w succinic acid and thereafter separating said solid containing more than 70% w/w succinic acid from the mixture. This process is not economically attractive.

We have now developed a simpler, more economic process for the recovery of succinic acid from its admixture with other carboxylic acids.

Accordingly, the present invention provides a process for the recovery of succinic acid from its admixture with other carboxylic acids which process comprises the steps of:

(A) heating the mixture of carboxylic acids under conditions of temperature and pressure and for a time sufficient to dehydrate at least a part of the succinic acid to succinic anhydride,

(B) separating by distillation the product from step (A) into a base fraction comprising carboxylic acids having a boiling point greater than that of succinic anhydride and a distillate fraction comprising succinic anhydride and succinic acid formed by hydrolysis of succinic anhydride by water resulting from dehydration in step (A), and

(C) recovering succinic acid from the distillate obtained in step (B).

In step (A) of the process the mixture of carboxylic acids is heated under conditions of temperature and pressure and for a time sufficient to dehydrate at least a part of the succinic acid to succinic anhydride. Preferably the conditions of temperature and pressure and the time are selected so as to maximise the dehydration of succinic acid to succinic anhydride. Conditions of temperature and pressure which effect dehydration of the succinic acid to succinic anhydride are of course inter-related. Typically, the temperature may be in the range from 180 to 210°C at a pressure of about 50 mm Hg. Determination of the optimum temperature for any given pressure will be well within the competence of the man skilled in the art, based on the foregoing. The time for which these conditions are maintained is preferably that required to dehydrate substantially all the succinic acid to succinic anhydride. Typically, under the aforesaid conditions the time may be up to about 4 hours.

Step (A) of the process is preferably carried out in the kettle of the distillation column used to effect step (B), though it may be carried out in a separate reactor if required.

In step (B) of the process the product from step (A) is separated by distillation into a base fraction comprising carboxylic acids having a boiling point greater than that of succinic anhydride and a distillate fraction comprising succinic anhydride and succinic acid formed by hydrolysis of succinic anhydride by water resulting from dehydration in step (A).

In step (C) of the process succinic acid is recovered from the distillate obtained in step (B). This may be effected without any intervening treatment of the distillate, suitably by crystallisation or precipitation followed by filtration or centrifugation. However, in a preferred embodiment of the present invention succinic acid is recovered indirectly from the distillate fraction obtained in step (B) by the steps of contacting the distillate fraction with a hydrolysing agent under conditions whereby succinic anhydride comprised in the distillate fraction is hydrolysed to succinic acid and thereafter recovering succinic acid from the hydrolysate.

A suitable hydrolysing agent is water. Suitable hydrolysis conditions comprise the use of an elevated temperature, up to but not including the boiling point of the hydrolysing agent under the prevailing pressure.

Succinic acid may suitably be separated from the hydrolysate by crystallisation. This may suitably be achieved by cooling the hydrolysate. Following crystallisation the succinic acid in crystalline form is recovered from the hydrolysate, suitably by filtration and/or centrifugation, preferably by centrifugation.

It is thereafter preferred to wash the recovered succinic acid with a suitable solvent, for example an aliphatic or aromatic hydrocarbon, an ester, an alcohol, a ketone or an aldehyde, or a mixture of the aforesaid solvents. The wash treatment may suitably be repeated one or more times, preferably recycling the solvent after each wash. Finally, it is preferred to dry the succinic acid.

In addition to succinic acid, the mixture of carboxylic acids may contain adipic acid and/or glutaric acid. Other acids which may be present in varying amounts include laevulinic acid, malonic acid and benzoic acid. Typically the mixture of carboxylic acids may be a by-product fraction derived from either the aforesaid cyclohexanol/cyclohexanone oxidation process or the aforesaid hydrocarbon oxidation process, as described in for example GB-A-743,989; GB-A-743,990; GB-A-771,991; GB-A-767,290 or GB-A-771,992.

If desired, the purity of the succinic acid recovered by the process of the invention may be further improved by, for example, recrystallisation and/or treatment with activated carbon.

The process may be operated batchwise or continuously, preferably continuously.

The invention will now be further illustrated by reference to the following Examples.

Procedure

0.25-0.75 kg samples of a mixture of carboxylic acids having the following composition:

| Component | % w/w |
|---|---|
| Malonic acid | 0.14 |
| Laevulinic acid | 6.6 |
| Succinic acid | 14.8 |
| Benzoic acid | 1.3 |
| Glutaric acid | 14.4 |
| Adipic acid | 4.1 |
| Water | 0.3 |
| Unidentified (esters, ketones aldehydes, lactones, resins, tars, etc). | 58.36 |

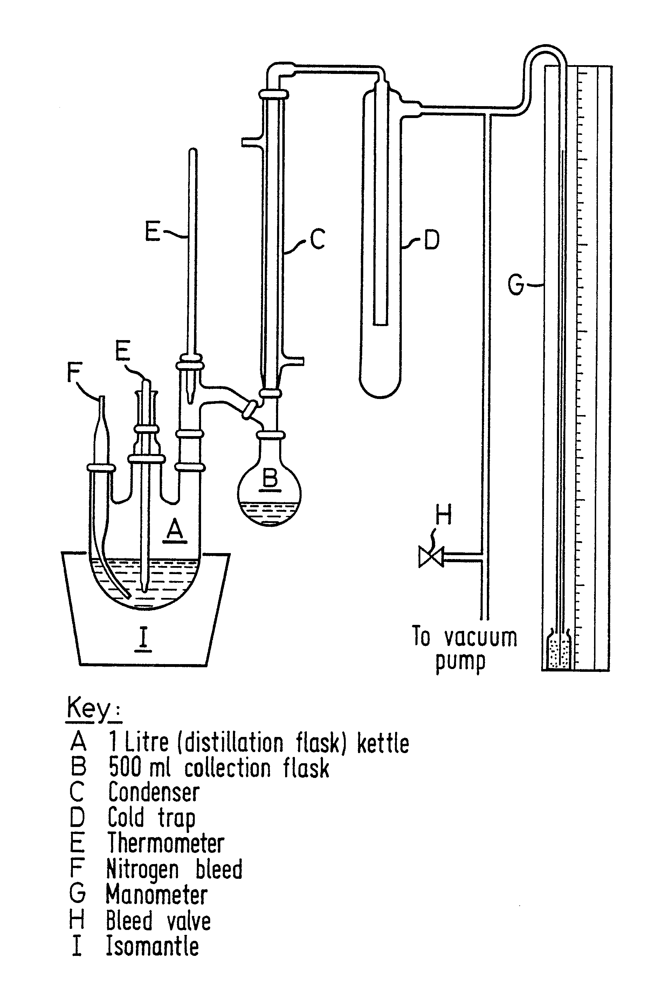

obtained as a residue fraction from the oxidation of a paraffinic hydrocarbon fraction to $C_1$ to $C_4$ monocarboxylic acids, were subjected to a simple distillation under vacuum (50 mm Hg) in a series of experiments in which the final kettle temperature was deliberately limited to specific values within the range 180-210°C. The distillation apparatus is shown in the accompanying Figure in which 1 (A) is a litre (distillation flask) kettle, 2 (B) is a 500 ml collection flask, 3 (C) is a condenser, 4 (D) is a cold trap, 5 (E) is a thermometer, 6 (F) is a nitrogen bleed, 7 (G) is a manometer, 8 (H) is a bleed valve and 9 (I) is an isomantle. The final kettle temperature was usually reached within 1-2 h and the residues were maintained at this temperature for a further 1-2h to complete the distillation.

The distillate obtained was mobile, clear and relatively light in colour (yellow or orange) and in most cases showed definite signs of solids precipitation at room temperature by the end of the distillation period. The total distillate was then heated for 1 hour at ca. 90°C in the presence of 10% wt. added water to hydrolyse any residual succinic anhydride back to the acid. During the heating-up period the initial solid redissolved. Towards the end of the hydrolysis period succinic acid began to crystallise from solution, and crystallisation was completed by cooling to room temperature over 1-2 hours.

The distillate was then filtered to recover crude succinic acid as a yellow, fairly coarsely crystalline filter cake, containing ca. 25-45% wt. mother liquor depending on the efficiency of filtration. (It has been found that a white 'crude' product can be obtained if this initial separation is effected by centrifugation rather than

3

by filtration). The filter cake was washed on the filter with approximately half its weight of DLE (a mixture of $C_4$-$C_7$ hydrocarbons, benzene, aldehydes, ketones, esters and alcohols), the filtrate being recycled as necessary to improve the washing efficiency. This wash procedure was then repeated a further twice (total solvent usage ca. 1.5 times weight of crude cake). The solvent wash completely removed the yellow colouration and, on subsequent drying at 80°C (usually overnight), a white free-flowing 'technical' grade of succinic acid was obtained.

## Results

The amount of distillate and the final yields of dry, technical grade succinic acid obtained at various distillation temperatures are summarised in the Table.

### TABLE

#### Succinic Acid Recovery - Effect of Distillation Temperature

#### at a Pressure of 50 mm Hg

Scale:　0.25-0.75 Kg charge

| Final Kettle Temperature °C | Total Distillation % w/w on charge | Succinic Acid Recovered (1) % w/w on charge | Average Concentration of Succinic Acid in Distillate % w/w | |
|---|---|---|---|---|
| | | | Recoverable | Total (2) |
| 180 | 10.1 | 1.0 | 9.9 | 17 |
| 190 | 28.7 | 4.2 | 14.6 | 21 |
| 190 | 31.3 | 5.1 | 16.2 | 23 |
| 190 | 30.5 | 5.6 | 18.3 | 25 |
| 190 | 34.4 | 5.0 | 14.5 | 21 |
| 200 | 43.3 | 9.1 | 21.0 | 27 |
| 200 | 41.0 | 7.9 | 19.3 | 26 |
| 210 | 51.1 | 11.6 | 22.7 | 29 |
| 210 | 51.1 | 11.8 | 23.1 | 29 |
| 210 | 50.9 | 10.2 | 20.0 | 26 |

NOTES:　(1)　Product yields determined after washing with (DLE), and oven drying to constant weight.

(2)　Assuming that solubility of succinic acid in distillate is ca. 8% w/w.

## Product Quality

The technical grade product achieved by washing the crude product with DLE and subsequent drying is a white free-flowing crystalline powder. It melts ca. 2-3°C lower than pure succinic acid and assays as 99.5 - 100% succinic acid equivalent by titration. Solution in water are clear and colourless. A purity of .96-99% is indicated by glc analysis, the main impurities being laevulinic acid and glutaric acid. There is no evidence of any deterioration in quality as the distillation temperature is increased to improve the recovery efficiency.

A single recrystallisation from water raises the melting point to the value expected for pure succinic acid and the 'purity' by glc analysis to ca 99.4-99.7% w/w.

## Claims

1 A process for the recovery of succinic acid from its mixture with other carboxylic acids which process comprises the steps of:

(A) heating the mixture of carboxylic acids under conditions of temperature and pressure and for a time sufficient to dehydrate at least a part of the succinic acid to succinic anhydride,

(B) separating by distillation the product from step (A) into a base fraction comprising carboxylic acids having a boiling point greater than that of succinic anhydride and a distillate fraction comprising succinic anhydride and succinic acid formed by hydrolysis of succinic anhydride by water resulting from dehydration in step (A), and

(C) recovering succinic acid from the distillate fraction obtained in step (B).

2 A process according to claim 1 wherein in step (A) the conditions of temperature and pressure and the time are selected so as to maximise the dehydration of succinic acid to succinic anhydride.

3 A process according to claim 2 wherein the temperature is in the range from 180 to 210°C and the pressure is about 50 mm Hg.

4 A process according to either claim 2 or claim 3 wherein the time for which the conditions are maintained is that required to dehydrate substantially all the succinic acid to succinic anhydride.

5 A process according to any one of claims 2 to 4 wherein the time is up to about 4 hours.

6 A process according to any one of the preceding claims wherein step (A) is carried out in the kettle of the distillation column used to effect step (B).

7 A process according to any one of the preceding claims wherein succinic acid is recovered from the distillate obtained in step (B) without any intervening treatment.

8 A process according to any one of claims 1 to 6 wherein succinic acid is recovered indirectly from the distillate fraction obtained in step (B) by the steps of contacting the distillate fraction with a hydrolysing agent under conditions whereby succinic anhydride comprised in the distillate fraction is hydrolysed to succinic acid and thereafter recovering succinic acid from the distillate.

9 A process according to claim 8 wherein the hydrolysing agent is water.

10 A process according to either claim 8 or claim 9 wherein hydrolysis conditions comprise the use of an elevated temperature up to but not including the boiling point of the hydrolysing agent under the prevailing pressure.

11 A process according to any one of claims 8 to 10 wherein succinic acid is separated from the hydrolysate by crystallisation and is thereafter recovered from the hydrolysate by centrifugation.

12 A process according to any one of the preceding claims wherein the recovered succinic acid is recrystallised and/or treated with activated carbon to improve the purity thereof.

13 A process according to any one of the preceding claims wherein the mixture of carboxylic acids contains, in addition to succinic acid, adipic acid and/or glutaric acid.

14 A process according to any one of claims 1 to 12 wherein the mixture of carboxylic acids is a by-product fraction derived from a cyclohexanol/cyclohexanone oxidation process.

15 A process according to any one of claims 1 to 12 wherein the mixture of carboxylic acids is a by-product fraction obtained from the liquid phase oxidation of paraffinic hydrocarbons with molecular oxygen to produce as the principal products lower carboxylic acids.

16 A process according to claim 1 substantially as hereinbefore described with reference to the Example.

Key:
A   1 Litre (distillation flask) kettle
B   500 ml collection flask
C   Condenser
D   Cold trap
E   Thermometer
F   Nitrogen bleed
G   Manometer
H   Bleed valve
I   Isomantle

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-2 971 010  (GILBY Jr. et al.)<br>* Claims 1-4; column 1, lines 37-46; columns 3-5, example 1; figures *<br>--- | 1-4,7-14 | C 07 C  55/10<br>C 07 C  51/44<br>C 07 C  51/42<br>C 07 C  51/573<br>C 07 C  51/56 |
| A | US-A-3 359 283  (CAMPBELL et al.)<br>* Claims 1-14; column 2, lines 36-45; figures *<br>----- | 1-4,14 | |

TECHNICAL FIELDS
SEARCHED (Int. Cl.4)

C 07 C  55/00
C 07 C  51/00

The present search report has been drawn up for all claims

| Place of search | Date of completion of the search | Examiner |
|---|---|---|
| THE HAGUE | 23-03-1988 | KLAG M.J. |

EPO FORM 1503 03.82 (P0401)